# EUROPEAN PATENT SPECIFICATION

(11) **EP 3 512 470 B1**
(45) Date of publication and mention of the grant of the patent: **01.11.2023**
(21) Application number: 16777916.4
(22) Date of filing: 16.09.2016
(51) Int. Cl.: A61F 5/14, A43B 7/14, A43B 17/00

(54) **ORTHOTIC INSERT FOR ALLEVIATING SEVER'S DISEASE**
ORTHOPÄDISCHE EINLAGE ZUR LINDERUNG DER SEVER-KRANKHEIT
INSERT ORTHÉTIQUE POUR SOULAGER LA MALADIE DE SEVER

(43) Date of publication of application: 24.07.2019
(73) Proprietor: Solemaids ApS, 8800 Viborg (DK)
(72) Inventor: ØLLGAARD, Per, 8800 Viborg (DK)
(74) Representative: Larsen & Birkeholm A/S
(86) International application number: PCT/EP2016/072000
(87) International publication number: WO 2018/050241

(56) References cited:
- EP-A1- 3 011 853
- WO-A1-94/19978
- US-A1- 2001 045 027

## Description

### Technical field of the invention

The present invention relates to Sever's Disease. More specifically, the invention relates to an orthotic insert for alleviating Sever's Disease.

### Background of the invention

Active children commonly experience the ache and debilitating effects of posterior heel pain as a result of Sever's Disease (calcaneal apophysitis). This disease in particular affects young athletes. Sever's Disease is a growth plate injury in which the Achille's Tendon puts excessive pressure on the posterior calcaneal apophysis (growth plate), causing inflammation and irritation of the apophysis. This can create great pain for young athletes.

The Foot, Volume 17, Issue 4, December 2007, Pages 178-183 states that taping the arch can be a very effective method in relieving symptoms in individuals with Sever's Disease. However, taping requires time and expertise that might not be available for young athletes at their athletic venue.

Insoles for a shoe are known from EP 3 011 853 A1 and US 2001/045027 A1. An orthotic device is known from WO 94/19978 A1.

### Summary of the invention

Hence, one object of the present invention is to provide an orthotic insert for relieving symptoms in individuals with Sever's Disease.

The invention is as defined in the appended claims and relates to an orthotic insert for children suffering from Sever's disease. The orthotic insert comprises:
- a shell plate, which has a length corresponding to the length of the user's foot; and
- a wedge attached to the top surface of the shell plate, and extending from a back end position on the shell plate corresponding to a position in front of the user's medial and lateral process of tuberosity, but in close proximity thereto, and towards a front end position on the shell plate corresponding to a position of the user's metatarsus;
   wherein the wedge has a front face portion and a rear face portion; wherein the rear face portion inclines upward from the shell plate, and wherein the front face portion declines downward from the front part of the rear face portion towards the shell plate;
   wherein the front face portion of the wedge is positioned such that a part of the user's foot corresponding to the calcaneal tubercle is resting thereon; and
- optionally, an insole covering a part of the top surface of the shell plate, the front face portion, and the rear face portion.

Orthotic inserts (into a shoe) are used to provide additional support for the user's foot, particularly in circumstances where the wearer requires a degree of bio-mechanical support and control.

The primary function of the orthotic insert according to the present invention is to relieve the pain in a person's heel as his or her foot strikes the ground during walking or running, and to provide sufficient rest to the apophysis in order to reduce or remove the inflammation therein.

This is done by raising the distal part of the calcaneus with a wedge. Thereby, the proximal part of the calcaneus, where the apophysis is present, is raised to such an extent that the proximal part of the user's heel only slightly touches the orthotic insert as his or her foot strikes the ground during walking or running. Hence, the impact loads imparted to the heel are in this way mainly transmitted to the distal part of the calcaneus. Preferably, the shell plate is formed to the contour of the sole portion of the user's foot. The material from which the shell plate is fabricated may be one of any number of thermoplastics or thermosetting plastics. Polyethylene, polypropylenes, acrylics, polycarbonates, ABS plastics, PVC plastics, polyesters, epoxy resins, and various laminated plastics may be used. A preferred composition for the shell plate is a thermoplastic co-polymer of polypropylene with about 15% polyethylene added.

A wedge is attached to the top surface of the shell plate. It extends from a position on the shell plate corresponding to a position in front of the user's medial and lateral process of tuberosity, but in close proximity thereto, and towards a position on the shell plate corresponding to a position of the user's metatarsus. It is crucial that the wedge will not interact with the user's medial and lateral process of tuberosity, since the impact loads imparted to the heel during exercise will otherwise mainly be transmitted to the proximal part of the calcaneus, where the apophysis is present.

Preferably, the wedge is formed to the contour of the sole portion of the user's foot.

In one or more embodiments, the wedge extends from a position on the shell plate corresponding to a position within the range of 1-30 mm in front of the user's medial and/or lateral process of tuberosity, and towards a position on the shell plate corresponding to a position of the user's metatarsus; such as within the range of 2-28 mm, e.g. within the range of 3-26 mm, such as within the range of 4-24 mm, e.g. within the range of 5-22 mm, such as within the range of 6-20 mm, e.g. within the range of 7-18 mm, such as within the range of 8-16 mm, e.g. within the range of 10-15 mm in front of the user's medial and lateral process of tuberosity, and towards a position on the shell plate corresponding to a position of the user's metatarsus.

In one or more embodiments, the wedge extends from a position on the shell plate corresponding to a position within the range of 1-30 mm in front of the user's medial and/or lateral process of tuberosity, and towards the front end (distal end) of the shell plate; such as within the range of 2-28 mm, e.g. within the range of 3-26 mm, such as within the range of 4-24 mm, e.g. within the range of 5-22 mm, such as within the range of 6-20 mm, e.g. within the range of 7-18 mm, such as within the range of 8-16 mm, e.g. within the range of 10-15 mm in front of the user's medial and lateral process of tuberosity, and towards the front end (distal end) of the shell plate.

According to the invention, the wedge extends from a position on the shell plate of 35-55 mm from the rear end of the shell plate, and towards a position on the shell plate corresponding to a position of the user's metatarsus, such as within the range of 37-53 mm, e.g. within the range of 39-51 mm, such as within the range of 41-49 mm, e.g. within the range of 43-47 mm, preferably within the range of 40-46 mm from the rear end of the shell plate, and towards a position on the shell plate corresponding to a position of the user's metatarsus.

According to the invention, the wedge extends from a position on the shell plate of 35-55 mm from the rear end of the shell plate, and towards the front end (distal end) of the shell plate, such as within the range of 37-53 mm, e.g. within the range of 39-51 mm, such as within the range of 41 - 49 mm, e.g. within the range of 43-47 mm, preferably within the range of 40-46 mm from the rear end of the shell plate, and towards the front end (distal end) of the shell plate.

The exact front end position of the wedge on the shell plate is of minor relevance, as long as the minimum length is enough to support the portion of the user's foot corresponding to the distal part of the calcaneus.

In one or more embodiments, the length of the wedge is within the range of 5-25 cm, such as within the range of 7-23 cm, e.g. within the range of 9-21 cm, such as within the range of 11-19 cm, e.g. within the range of 13-17 cm, preferably within the range of 5-15 cm.

The wedge has a front face portion and a rear face portion, facing the user's foot. The rear face portion inclines upward from the shell plate, and the front face portion declines downward from the front part of the rear face portion towards the shell plate. In one or more embodiments, a part of the front face portion bordering to the rear face portion is horizontal.

The front face portion of the wedge is positioned such that a part of the user's foot corresponding to the calcaneal tubercle is resting thereon. Hence, the wedge material below the front face portion will direct/transmit the impact loads to the heel during exercise to the distal part of the calcaneus.

In one or more embodiments, the front face portion of the wedge comprises a plurality of sub-portions with different angles of declination. In one or more embodiments, a sub-portion of the front face portion positioned closer to the rear face portion than a neighbouring sub-portion has a lower angle of declination than said neighbouring sub-portion. Sub-portions with different angles of declination are present for better alignment with the contour of the sole portion of the user's foot.

In one or more embodiments, the inclination angle of the rear face portion is within the range of 35-75 degrees, such as within the range of 40-70 degrees, e.g. within the range of 45-65 degrees, such as within the range of 50-60 degrees, preferably within the range of 45-60 degrees.

In one or more embodiments, the rear face portion inclines in a convex curve upward from the shell plate. The convex curve is present for better alignment with the contour of the sole portion of the user's foot.

In order to raise the raise the distal part of the calcaneus to such an extent that the proximal part of the user's heel only slightly touches the orthotic insert as his or her foot strikes the ground during walking or running, the minimum thickness of the part of the front face portion bordering to the rear face portion should be of a reasonable thickness.

In one or more embodiments, the thickness of the part of the front face portion bordering to the rear face portion is within the range of 5-40 mm, e.g. within the range of 7-16 mm, such as within the range of 12-38 mm, e.g. within the range of 14-36 mm, such as within the range of 16-34 mm, e.g. within the range of 18-32 mm, such as within the range of 20-30 mm, e.g. within the range of 22-28 mm, preferably within the range of 12-16 mm. The inventor has found that the debilitating effects of posterior heel pain as a result of Sever's Disease heel may be alleviated or even removed when the wedge is made of a compressible material having a Shore A-scale hardness measured according to ASTM D2240-97 within the range of 14-35. This range is surprisingly narrow. If the compressible material has a Shore A-scale hardness measured according to ASTM D2240-97 below 14, the inventor has found that the user will still experience a pain localized to the posterior part of the heel that prevent the user from running. Without being bound by any particular theory, it is thought that the posterior part of the user's heel is forced too deep into the orthotic insert, resulting in the anterior part of the user's heel absorbing much more of the impact loads imparted to the heel when the user is running.

If the compressible material has a Shore A-scale hardness measured according to ASTM D2240-97 above 35, the user will also experience a pain localized to the posterior heel that prevent the user from running. This is indeed surprising in the hardness area around 36-60 (Shore A-scale), since the material is still relatively soft.

In one or more embodiments, the wedge is made of a compressible material having a Shore A-scale hardness measured according to ASTM D2240-97 within the range of 14-35, such as within the range of 16-33, e.g. within the range of 18-31, such as within the range of 20-29, e.g. within the range of 22-27, such as within the range of 24-26, preferably within the range of 22-30.

Examples of material(s) for the wedge can comprise thermoplastic elastomers, polyester, polypropylene, polyethylene, polyurethane, polyurea, cellular urethane, polystyrene, latex, nylon, carbon or glass fibres with thermosetting or thermoplastic resins, plastizote, ethylene vinyl acetate (EVA) foams, expanded vinyl foam, flocked vinyl film, coagulated polyurethane, latex foam on scrim, supported polyurethane foam, polyurethane film, styrene-butadiene-rubber, acrylonitrile-butadiene, acrylonitrile terpolymers and copolymers, styrene-butadienestyrene, acrylonitrile-butadiene-styrene, polyvinyl chloride (PVC), ethylenepropylene rubber, silicone rubber (SiR), silicone elastomer, acrylic rubber (ACM), butadiene rubber (BR), butyl rubber (IIR), chlorosulfonated polyethylene (CSM)/hypalonthylene propylene diene monomer (EPM, EPDM), fluoroelastomers (FKM), isoprene rubber (IR), nitrile rubber (NBR), perfluoroelastomer (FFKM), polychloroprene (CR), polysulfide rubber (PSR), styrene butadiene rubber (SBR), or any combination thereof.

The wedge may comprise a foam, a rubber, or any combination thereof. The foam can comprise an open cell foam, a closed cell foam, or both.

In one or more embodiments, the wedge is made of an open cell and/or closed cell foam material. Preferably the wedge is made of a closed cell foam material, since moist may enter the open cell foam material, and thereby change its hardness (Shore A).

In one or more embodiments, wedge is made of a monolithic material.

### Brief description of the figures

Figure 1 shows an orthotic insert in accordance with various embodiments of the invention; and
Figure 2 shows the calcaneus of a user's right foot (medial view) on top of an orthotic insert in accordance with various embodiments of the invention.

### Detailed description of the invention

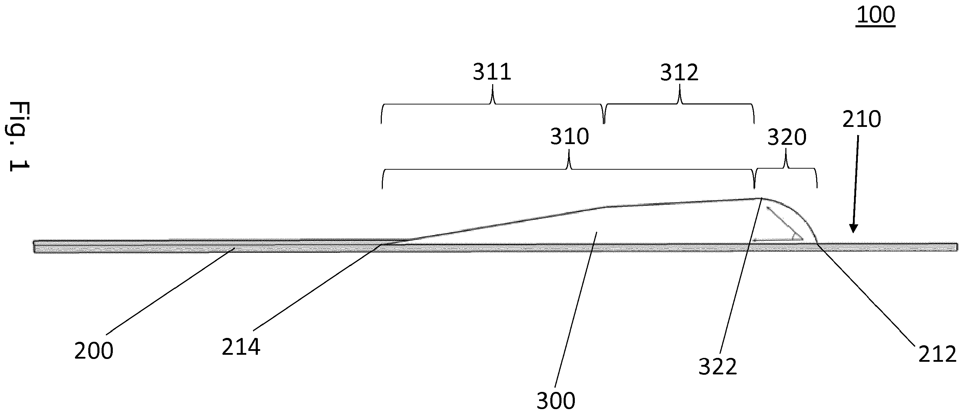

Referring to Figure 1, the general scheme of the invention is shown. Figure 1 shows an orthotic insert 100. The orthotic insert 100 comprises a shell plate 200 and a wedge 300.

The shell plate 200 has a length corresponding to the length of the user's foot.

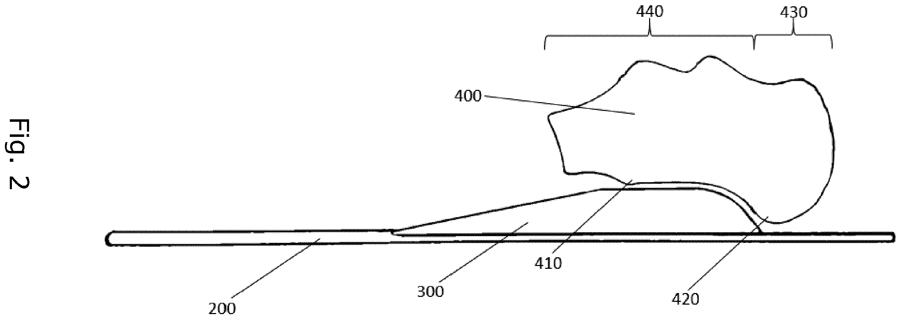

The wedge 300 is attached to the top surface 210 of the shell plate 200. It extends from a back end position 212 on the shell plate 200 corresponding to a position in front of the user's medial and lateral process of tuberosity, and towards a front end position 214 on the shell plate 200 corresponding to a position of the user's metatarsus. The position of the wedge in relation to the calcaneus is shown in Figure 2. It is crucial that the wedge will not interact with the user's medial and lateral process of tuberosity, since the impact loads imparted to the heel during exercise will otherwise mainly be transmitted to the proximal part of the calcaneus, where the apophysis is present.

The exact back end position 212 on the shell plate 200 varies depending on the size of the user's foot. The children suffering from Sever's disease are normally of the age of 8-14 years, and with EU foot sizes of 32-43. For this group of patients, the back end position 212 of the wedge 300 is situated 35-55 mm from the rear end of the shell plate 200.

The wedge 300 has a front face portion 310 and a rear face portion 320. The rear face portion 320 inclines upward from the shell plate 200; and the front face portion 320 declines downward from the front part 322 of the rear face portion 320 towards the shell plate 200.

The front face portion 320 of the wedge 300 is positioned such that a part of the user's foot corresponding to the calcaneal tubercle 410 is resting thereon. In Figure 1, the front face portion 310 of the wedge comprises two sub-portions (311, 312) with different angles of declination.

Figure 2 shows the calcaneus 400 of a user's right foot (medial view) on top of an orthotic insert 100 in accordance with various embodiments of the invention. The wedge 300 is attached to the top surface 210 of the shell plate 200. It extends from a back end position 212 on the shell plate 200 corresponding to a position in front of the user's medial 420 and lateral (not shown) process of tuberosity, and towards a front end position 214 on the shell plate 200 corresponding to a position of the user's metatarsus. The exact front end position 214 of the wedge 300 on the shell plate 200 is of minor relevance, as long as the minimum length of the wedge 300 is enough to support the portion of the user's foot corresponding to the distal part 440 of the calcaneus 400.

### References

- 100: Orthotic insert
- 200: Shell plate
- 210: Top surface
- 212: Back end position
- 214: Front end position
- 300: Wedge
- 310: Front face portion
- 311: Sub-portion of front face portion
- 312: Sub-portion front face portion
- 320: Rear face portion
- 322: Front part of rear face portion
- 400: Calcaneus
- 410: Calcaneal tubercle
- 420: Medial process of tuberosity
- 430: Proximal part of Calcaneus
- 440: Distal part of Calcaneus

## Claims

1. An orthotic insert (100) for children suffering from Sever's disease, said insert (100) comprising:
- a shell plate (200), which has a length corresponding to the length of the user's foot; and
- a wedge (300) attached to the top surface (210) of the shell plate (200), and extending from a back end position (212) on the shell plate (200) corresponding to a position in front of the user's medial (420) and lateral process of tuberosity, but in close proximity thereto, and towards a front end position (214) on the shell plate (200) corresponding to a position of the user's metatarsus;
wherein the wedge (300) has a front face portion (310) and a rear face portion (320); wherein the rear face portion (320) inclines upwards from the back end position (212) on shell plate (200) to the front part (322), and wherein the front face portion (310) declines downward from the front part (322) of the rear face portion (320) towards the front end position (214) on the shell plate (200);
wherein the front face portion (310) of the wedge (300) is positioned such that a part of the user's foot corresponding to the calcaneal tubercle (410) is resting thereon; **characterized in that** the wedge extends from a position on the shell plate of 35-55 mm from the rear end of the shell plate, and towards the front end of the shell plate, wherein the wedge (300) is shaped such that the proximal part of the user's calcaneus (400), where the apophysis is present, is raised to such an extent that the proximal part of the user's heel only slightly touches the orthotic insert (100) as his or her foot strikes the ground during walking or running.

2. An orthotic insert (100) according to claim 1, wherein the inclination angle of the rear face portion (320) is within 45-60 degrees.

3. An orthotic insert (100) according to any one of the claims 1-2, wherein the rear face portion (320) inclines in a convex curve upward from the shell plate (200).

4. An orthotic insert (100) according to any one of the claims 1-3, wherein the thickness of the part of the front face portion bordering to the rear face portion (320) is within the range of 6-30 mm.

5. An orthotic insert (100) according to any one of the claims 1-4, wherein the wedge (300) is made of a compressible material having a Shore A-scale hardness measured according to ASTM D2240-97 within the range of 20-35.

6. An orthotic insert (100) according to any one of the claims 1-5, wherein the front part (322) of the rear face portion (320) is positioned on the shell plate (200) corresponding to a position beneath the distal part (440) of the user's Calcaneus.

7. An orthotic insert (100) according to any one of the claims 1-6, wherein the front face portion (310) of the wedge (300) comprises a plurality of sub-portions (311, 312) with different angles of declination.

8. An orthotic insert (100) according to claim 7, wherein a sub-portion (312) of the front face portion (310) positioned closer to the rear face portion (320) than a neighbouring sub-portion (311) has a lower angle of declination than said neighbouring sub-portion (311).

## Patentansprüche

1. Orthopädische Einlage (100) für Kinder, die an der Sever-Krankheit leiden, wobei die Einlage (100) umfasst:
- eine Schalenplatte (200), die eine Länge aufweist, die der Länge des Fußes des Benutzers entspricht; und
- einen Keil (300), der an der oberen Fläche (210) der Schalenplatte (200) angebracht ist und sich von einer hinteren Endposition (212) auf der Schalenplatte (200), die einer Position vor dem Processus medialis (420) und dem Processus lateralis des Fersenhöckers des Benutzers entspricht, jedoch in unmittelbarer Nähe dazu, und zu einer vorderen Endposition (214) auf der Schalenplatte (200) hin, die einer Position des Mittelfußes des Benutzers entspricht, erstreckt;
wobei der Keil (300) einen Stirnflächenabschnitt (310) und einen Rückflächenabschnitt (320) aufweist; wobei der Rückflächenabschnitt (320) von der hinteren Endposition (212) auf der Schalenplatte (200) nach oben zum Vorderteil (322) geneigt ist und wobei der Stirnflächenabschnitt (310) vom Vorderteil (322) des Rückflächenabschnitts (320) zur vorderen Endposition (214) auf der Schalenplatte (200) hin nach unten abfällt;
wobei der Stirnflächenabschnitt (310) des Keils (300) so positioniert ist, dass ein dem Fersenbeinhöcker (410) entsprechender Teil des Fußes des Benutzers darauf ruht; **dadurch gekennzeichnet, dass** sich der Keil von einer Position auf der Schalenplatte von 35-55 mm vom hinteren Ende der Schalenplatte und zum vorderen Ende der Schalenplatte hin erstreckt, wobei der Keil (300) so geformt ist, dass der proximale Teil des Fersenbeins (400) des Benutzers, wo die Apophyse vorhanden ist, so weit angehoben wird, dass der proximale Teil der Ferse des Benutzers die orthopädische Einlage (100) nur leicht berührt, wenn sein Fuß beim Gehen oder Laufen auf den Boden tritt.

2. Orthopädische Einlage (100) nach Anspruch 1, wobei der Neigungswinkel des Rückflächenabschnitts (320) innerhalb von 45-60 Grad liegt.

3. Orthopädische Einlage (100) nach einem der Ansprüche 1-2, wobei der Rückflächenabschnitt (320) in einer konvexen Kurve von der Schalenplatte (200) nach oben geneigt ist.

4. Orthopädische Einlage (100) nach einem der Ansprüche 1-3, wobei die Dicke des Teils des Stirnflächenabschnitts, der an den hinteren Flächenabschnitt (320) grenzt, im Bereich von 6-30 mm liegt.

5. Orthopädische Einlage (100) nach einem der Ansprüche 1-4, wobei der Keil (300) aus einem komprimierbaren Material hergestellt ist, das eine Härte der Shore-A-Skala, gemessen gemäß ASTM D2240-97, im Bereich von 20-35 aufweist.

6. Orthopädische Einlage (100) nach einem der Ansprüche 1-5, wobei der Vorderteil (322) des Rückflächenabschnitts (320) auf der Schalenplatte (200) entsprechend einer Position unter dem distalen Teil (440) des Fersenbeins des Benutzers positioniert ist.

7. Orthopädische Einlage (100) nach einem der Ansprüche 1-6, wobei der Stirnflächenabschnitt (310) des Keils (300) eine Vielzahl von Unterabschnitten (311, 312) mit unterschiedlichen Deklinationswinkeln umfasst.

8. Orthopädische Einlage (100) nach Anspruch 7, wobei ein Unterabschnitt (312) des Stirnflächenabschnitts (310), der näher als ein benachbarter Unterabschnitt (311) am Rückflächenabschnitt (320) positioniert ist, einen geringeren Deklinationswinkel als der benachbarte Unterabschnitt (311) aufweist.

## Revendications

1. Insert orthétique (100) pour enfants souffrant de la maladie de Sever, ledit insert (100) comprenant :
- une plaque de coque (200), qui a une longueur correspondant à la longueur du pied de l'utilisateur ; et
- une cale (300) fixée à la surface supérieure (210) de la plaque de coque (200), et s'étendant depuis une position d'extrémité arrière (212) sur la plaque de coque (200) correspondant à une position devant la médiane de l'utilisateur (420) et le processus latéral de la tubérosité, mais à proximité immédiate de celui-ci, et vers une position d'extrémité avant (214) sur la plaque de coque (200) correspondant à une position du métatarse de l'utilisateur ; dans lequel la cale (300) a une partie de face avant (310) et une partie de face arrière (320) ; dans lequel la partie de face arrière (320) s'incline vers le haut depuis la position d'extrémité arrière (212) sur la plaque de coque (200) jusqu'à la partie avant (322), et dans lequel la partie de face avant (310) décline vers le bas depuis la partie avant (322) de la partie de face arrière (320) vers la position d'extrémité avant (214) sur la plaque de coque (200) ;
dans lequel la partie de face avant (310) de la cale (300) est positionnée de sorte qu'une partie du pied de l'utilisateur correspondant au tubercule calcanéen (410) repose dessus ; **caractérisé en ce que** la cale s'étend depuis une position sur la plaque de coque de 35 à 55 mm depuis l'extrémité arrière de la plaque de coque, et vers l'extrémité avant de la plaque de coque, dans lequel la cale (300) est conformée de sorte que la partie proximale du calcanéum (400) de l'utilisateur, où l'apophyse est présente, est soulevée à un point tel que la partie proximale du talon de l'utilisateur ne touche que légèrement l'insert orthétique (100) lorsque son pied touche le sol pendant la marche ou la course.

2. Insert orthétique (100) selon la revendication 1, dans lequel l'angle d'inclinaison de la partie de face arrière (320) est compris entre 45 et 60 degrés.

3. Insert orthétique (100) selon l'une quelconque des revendications 1 à 2, dans lequel la partie de face arrière (320) s'incline selon une courbe convexe vers le haut à partir de la plaque de coque (200).

4. Insert orthétique (100) selon l'une quelconque des revendications 1 à 3, dans lequel l'épaisseur de la partie de la partie de face avant bordant la partie de face arrière (320) est dans la plage de 6 à 30 mm.

5. Insert orthétique (100) selon l'une quelconque des revendications 1 à 4, dans lequel la cale (300) est constitué d'un matériau compressible ayant une dureté Shore A mesurée selon la norme ASTM D2240-97 dans la plage de 20 à 35.

6. Insert orthétique (100) selon l'une quelconque des revendications 1 à 5, dans lequel la partie avant (322) de la partie de face arrière (320) est positionnée sur la plaque de coque (200) correspondant à une position sous la partie distale (440) du calcanéum de l'utilisateur.

7. Insert orthétique (100) selon l'une quelconque des revendications 1 à 6, dans lequel la partie de face avant (310) de la cale (300) comprend une pluralité de sous-parties (311, 312) avec différents angles de déclinaison.

8. Insert orthétique (100) selon la revendication 7, dans lequel une sous-partie (312) de la partie de face avant (310) positionnée plus près de la partie de face arrière (320) qu'une sous-partie voisine (311) a un angle de déclinaison inférieur par rapport à ladite sous-partie voisine (311).
